# EUROPEAN PATENT SPECIFICATION

(11) **EP 4 463 095 B1**
(45) Date of publication and mention of the grant of the patent: **04.02.2026**
(21) Application number: 23719557.3
(22) Date of filing: 30.03.2023
(51) Int. Cl.: A61B 46/10

(54) **DRAPE CONNECTION WITH ALIGNMENT FEATURE FOR SURGICAL SLUSH MACHINE**
OP-ABDECKUNGSVERBINDUNG MIT AUSRICHTUNGSFUNKTION FÜR EINE CHIRURGISCHE SLUSH-MASCHINE
CONNEXION DE CHAMP AVEC ÉLÉMENT D'ALIGNEMENT POUR MACHINE CHIRURGICALE À GRANITÉ

(30) Priority: 13.02.2023 US 202318168208
(43) Date of publication of application: 20.11.2024
(73) Proprietor: Medline Industries, LP, Northfield, IL 60093 (US)
(72) Inventor: WHITE, Tracy, Cumming, Georgia 30028 (US); MATTHEWS, Steffanie, Thompson, Cumming, Georgia 30041 (US); HENDRIX, Heidi, Frances, Atlanta, Georgia 30318 (US); GOLENBERG, Nurit, Atlanta, Georgia 30324 (US)
(74) Representative: Lewis Silkin LLP
(86) International application number: PCT/US2023/016927
(87) International publication number: WO 2024/172824

(56) References cited:
- US-A- 5 502 980
- US-A1- 2014 226 434
- US-B1- 7 350 373

## Description

### RELATED APPLICATIONS

This application claims the benefit of U.S. Patent Application No. 18/168,208, filed February 13, 2023.

### TECHNICAL FIELD

This disclosure relates to systems for generating surgical slush, including sterile drapes and connector systems for connecting a sterile drape to a surgical slush machine.

### BACKGROUND

Surgical fluid is used during a variety of different medical procedures. For example, saline is often used during surgery to irrigate the site of operation. The saline can be poured on the site of surgery to flush the region of blood and other bodily matter, providing the clinician with a clear view of the region being operated upon and clean surfaces for performing the operation. As another example, surgical fluid may be frozen into a slush that is introduced into a particular region of the body. The slush can provide localized hypothermia therapy, cooling the region of the body or organ around which the slush is placed. This can be useful to temporarily reduce the amount of oxygenated blood needed by the body, such as in emergencies like cardiac arrest or severe head trauma.

A surgical slush machine may include a basin into which an operator pours surgical fluid, such as saline, that is subsequently cooled in the basin to form a slush. Before introducing the surgical fluid into the basin, the operator may cover the basin with a disposable drape to form sterile barrier between the basin and the surgical fluid. The basin can be cooled to a level sufficient to freeze the surgical fluid poured into the drape-covered basin. In practice, the frozen surgical liquid may tend to attach to sides of the drape receptacle in large clumps or pieces rather than automatically collecting within the drape receptacle interior. To avoid this problem and to help form a slush instead of clumps of surgical fluid, the surgical slush machine may be configured to manipulate the drape relative to the basin wall. For example, the bottom of the drape may be reciprocated up and down to loosen attached pieces of frozen saline from the sides of the drape, causing the released material to fall into off the sides of the drape as slush.
US5502980A discloses a sterile drape assembly including a sheet of sterile drape material bonded to a disk-like member. The drape establishes a sterile field atop a surgical slush machine and is conformable to a cooling basin to provide a drape container impervious to the sterile slush medium.
US7350373B1 discloses a surgical drape including a disk member for removable attachment to a thermal treatment system dislodgment mechanism. The mechanism manipulates the drape to dislodge congealed sterile liquid adhered to the sides of a container formed by the drape within a thermal treatment system basin. The disk member includes a base and a cover, where the cover is disposed on the top portion or sterile surface of the drape.
US20140226434A1 discloses an apparatus for connecting a sterile drape to an actuator of a surgical slush machine. The apparatus is an agitator that includes a support member and a latch. The sterile drape is attached to one surface of the support member and the latch extends from the opposite surface of the support member. The support member is configured to flex and bend when the agitator is operated by the actuator and the drape contains a solution to be made into slush.

### SUMMARY

The present invention relates to a drape for a surgical slush system as defined in claim 1. Further embodiments of the invention are defined in the dependent claims. In general, this disclosure is directed to devices, systems, and techniques associated with generating surgical slush, including sterile drapes and connector systems for connecting a sterile drape to a surgical slush machine. In some examples, a surgical slush system includes a basin configured to receive and hold a surgical fluid and a cooling device thermally coupled to the basin to cool surgical fluid in the basin to generate a surgical slush. The system can include and/or be configured to receive a sterile surgical drape. In use, the surgical drape can be positioned in the basin to separate the surgical fluid and the surgical slush from the wall of the basin itself. To help form surgical slush in the draped basin and prevent the accumulation of clumps of agglomerated frozen surgical fluid on the sidewalls of the draped basin, the surgical slush system can include a piston. The piston can move relative to the sidewall of the basin, e.g., causing the drape placed in the basin to periodically flex and cause frozen surgical fluid on the sidewalls of the drape to slough off as slush.

In practice, it can be challenging to form an appropriate interface between the piston of the surgical slush machine and the drape placed over the basin of the surgical slush machine. If the piston is not connected to the drape, the drape may not move and flex in a repeatable, controllable pattern. This can cause frozen fluid buildup on the sidewalls of the drape and inconsistent operation of the slush machine. For these and other reasons, it is desirable that the piston be mechanically connected to the drape placed over the basin. However, if the drape becomes rigid and/or overweight because of excessive frozen material buildup during extended operation, the drape may not flex sufficiently in response to movement of the piston. If the piston is rigidly connected to the drape in these situations, the piston may not be allowed to move through its full ranges of intended travel. This can cause the damage to the surgical slush system, such as causing the piston motor to burnout.

In accordance with some examples of the present disclosure, however, a surgical slush system is described that includes a connection between a piston associated with the surgical slush machine and drape placed over a basin of the surgical slush machine. The piston can include a drape engagement member configured to engage with an underside of the drape. In some examples, the drape engagement member is positioned on the end of the piston and includes one or more alignment members. The drape can carry a corresponding piston attachment member configured to engage with the drape engagement member carried by the piston. In some example, the piston attachment member carried by the drape defines a cavity configured to be positioned over the drape engagement member carried by the piston, including the one or more alignment members.

For example, in implementations, the piston attachment member carried by the drape includes one or more cutouts corresponding to the one or more alignment members provided by the drape engagement member carried by the piston. In use, an operator can insert the piston attachment member carried by the drape over the drape engagement member carried by the piston, positioning the one or more alignment members on the drape engagement member carried by the piston in the one or more cutouts piston attachment member carried by the drape. Configuring the piston with one or more alignment members may be useful to help rotationally align and/or interlock the piston relative to the drape. For example, the positioning of the one or more alignment members relative to one or more corresponding cutouts can set the relative rotational alignment of the piston to the drape. This can help ensure that the drape is in a proper rotational alignment to the piston, e.g., when the two components are engaged and/or in use, and inhibit relative rotation or slipping between the two components during use.

In some configurations, the connection between the piston associated with the surgical slush machine and the drape inserted over the basin of the surgical slush machine is a breakaway connection that allows the piston to separate from the drape if a force threshold is exceeded. The connection between the piston and the drape may be sufficiently strong and/or rigid to inhibit separation between the piston and the drape during normal operation of the surgical slush machine (e.g., resist separation between the piston and the drape within the range of force expected to be encountered during normal operation of the surgical slush machine). However, the connection between the piston and the drape may be sufficiently weak and/or flexible to allow the piston to separate from the drape if a force threshold is exceeded (e.g., a force threshold that, if the piston and the drape remained connected above the threshold, may have a tendency to damage the surgical slush machine). For example, the connection between the piston and the drape may be configured to separate if excessive ice buildup on the drape would otherwise limit travel of the piston and potentially cause burnout of a motor driving the piston. The disengagement force between the piston and drape may be controlled by controlling the configuration of the drape engagement member carried by the piston and the piston attachment member carried by the drape including, in some examples, the configuration of the one or more alignment pins and one or more corresponding cutouts to receive the alignment pins.

In one example, a surgical slush system is described that includes a basin configured to receive and hold a surgical fluid and a thermal treatment device thermally coupled to the basin and configured to cool the surgical fluid to generate a surgical slush. The example system also includes a surgical drape and a piston. The surgical drape is configured to be positioned in the basin to separate the surgical fluid and the surgical slush from the basin. The surgical drape includes a top side configured to face away from the basin and a bottom side configure to face the basin, and the surgical drape also includes a piston attachment member on the bottom side. The piston has a terminal end projecting into the basin and is operable to move relative to the basin. The terminal end of the piston defines a drape engagement member. According to the example, the drape engagement member of the piston includes at least one sidewall defining a perimeter of a drape engagement plate and at least one alignment member extending outwardly from the drape engagement plate. The example also specifies that the piston attachment member on the bottom side of the surgical drape defines a receiving cavity configured to be positioned over the drape engagement plate and the at least one alignment member of the piston.

In another example, a drape for a surgical slush system is described. The drape includes a basin portion configured to be inserted into a basin of a surgical slush system. The basin portion includes a top side configured to face away from the basin of the surgical slush system and a bottom side configure to face the basin of the surgical slush system. The drape also includes a flexible side sheet extending about the basin portion and configured be positioned draping side portions of the surgical slush system. The drape further includes a piston attachment member on the bottom side of the basin portion. According to the example, the piston attachment member includes at least one downwardly extending sidewall defining a perimeter of a receiving cavity and at least one cutout extending through the downwardly extending sidewall, the cutout being configured to receive an alignment member of the surgical slush system.

In another example, a method of draping a surgical slush system is described. The method includes positioning a basin portion of a surgical drape in a basin of a surgical slush system, where the surgical drape includes a piston attachment member defining a receiving cavity on a bottom side of the surgical drape and the surgical slush system includes a piston carrying a drape engagement plate and at least one alignment member extending outwardly from the drape engagement plate. The example method further involves pressing the piston attachment member of the surgical drape downwardly on the drape engagement plate and the at least one alignment member of the surgical slush system.

The details of one or more examples are set forth in the accompanying drawings and the description below. Other features, objects, and advantages will be apparent from the description and drawings, and from the claims.

### BRIEF DESCRIPTION OF DRAWINGS

FIGS. 1 and 2 are different perspective views of an example system for thermally treating a surgical fluid
FIG. 3 is a functional block diagram illustrating components of an example configuration of the thermal treatment system of FIGS. 1 and 2.
FIG. 4 is a perspective view of one example configuration of a drape that can be used on a thermal treatment system and/or surgical slush system.
FIG. 5 is an exploded perspective view of an example configuration of a connection system that can be used between a drape and a piston of surgical slush system.
FIGS. 6A and 6B are perspective and top views, respectively, of an example configuration of a drape engagement member that can be used on a piston of a surgical slush machine.
FIGS. 7A and 7B are different perspective views illustrating a bottom side of an example piston attachment member.
FIG. 8 is an expanded view of an example cutout configuration that can be used for the one or more cutouts on a piston attachment member.
FIG. 9 is a perspective view illustrating a bottom side of an example configuration of a piston attachment member.
FIG. 10 is an expanded view of a portion of an example configuration a piston attachment member.
FIG. 11 is a perspective view illustrating a bottom side of another example configuration of a piston attachment member.
FIG. 12 is an expanded view of the cutout configuration of the example piston attachment member shown in FIG. 11.
FIGS. 13A-13C illustrate assembly of an example piston attachment member according to FIG. 12 to an example drape engagement member.

### DETAILED DESCRIPTION

This disclosure is generally directed to surgical slush machines and associated sterile drapes and connector systems for connecting a sterile drape to a surgical slush machine. In some examples, a surgical slush device includes a basin configured to receive and hold a surgical fluid and a cooling device thermally coupled to the basin to cool surgical fluid in the basin to generate a surgical slush. The surgical slush device can include a piston that actuates relative to the basin, e.g., moving up and down in a reciprocating motion. The height of the piston can change relative to the wall surface of the basin during motion to provide a relative offset of changing dimension between the piston and wall surface during motion.

A sterile drape can be connected to the piston, with drape covering the basin to provide a sterile barrier between the basin and surgical fluid placed in the basin and/or slush generated by freezing the surgical fluid. The sterile drape can include a connector on the bottom side of the drape that engages, directly or indirectly, with the piston. The sterile drape can also include a region configured to be draped over and cover the basin, which may be a flexible sheeting region and/or a formed (e.g., shaped) basin region (e.g., rigid polymeric region, thermoformed polymeric region). In either case, the drape can carry a connector that interfaces with the piston to form a mechanical connection between the drape and the piston. As a result, when the piston actuates relative to the wall surface of the basin, the drape connected the piston correspondingly moves in proportion to the movement of the piston. This can cause surgical fluid freezing on the drape in the basin to disengage from the surface of the drape (in response to movement of the drape caused by movement piston) rather than building up excessively to form clumps of ice less desirable than velvety soft slush in the operating room.

In some examples, the connection system between the piston and the drape includes one or more alignment features that help align positioning of the drape when connected to the piston and/or help connect the drape to the piston. For example, the piston may define one or more alignment members extending outwardly from a plate on the end the piston. The drape can have an attachment member on the bottom side of the drape that includes a receiving cavity configured to receive the one or more alignment members and/or the plate on the end of the piston. For example, the drape may include an attachment member formed by a sidewall configured to be positioned down over the side of the plate on the end of the piston. The sidewall can include one or more cutouts corresponding to the one or more alignment members on the piston. Upon attaching the drape to the piston, the one or more alignment members can be positioned into the one or more cutouts on the attachment member carried by the drape.

In some configurations, the attachment member carried by the drape is configured to be rotationally interlocked with the piston. For example, to attach the drape to the piston, the attachment member carried by the drape may be positioned down on top of the plate carried by the piston (e.g., press down on top of the plate). The attachment member can include one or more vertical cutouts into which the one or more alignment members extending outwardly from the plate can be introduced. After pressing the attachment member down on the plate, the drape may be rotated into a locking position in which the one or more alignment members rotate from a vertical cutout in the attachment member into an intersecting horizontal cutout. This can position the one or more alignment members between upper and lower sidewall portions of the attachment member. The attachment member carried by the drape can have a variety of additional or different configurations as described herein.

By configuring the piston with one or more alignment members configured to be engaged by the drape, the drape may be connected with the piston at a particular alignment location defined by the one or more alignment members. This can be beneficial, for example, to help prevent the drape from being twisted during engagement with the piston and/or during movement of the piston, which may otherwise affect the freezing of surgical fluid on the surface of the drape and/or the structural integrity of the drape.

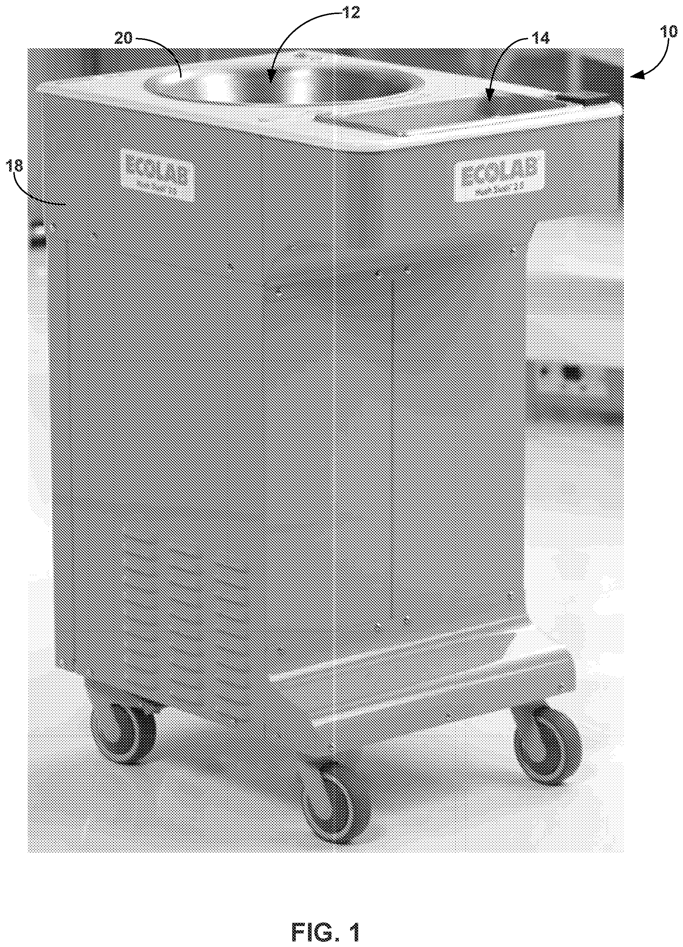

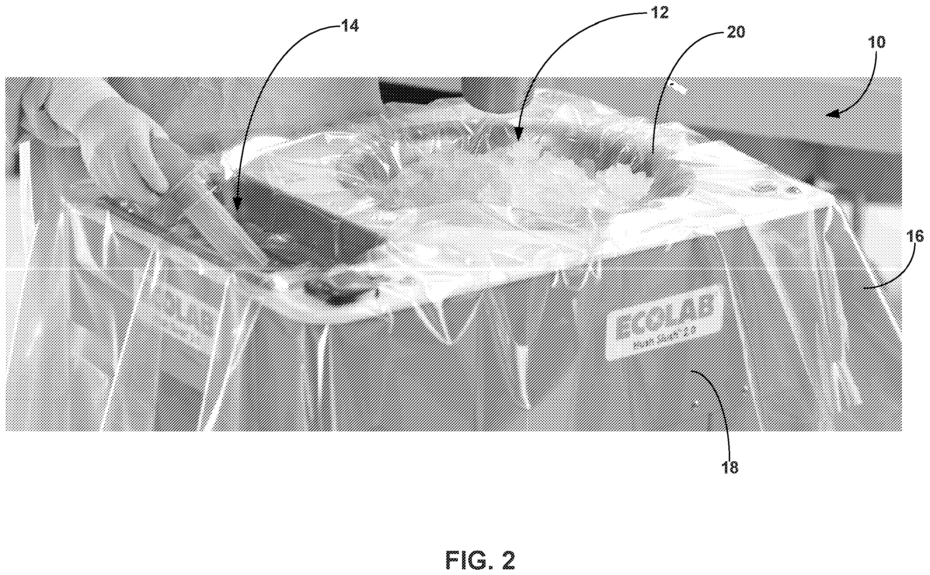

FIGS. 1 and 2 are different perspective views of an example system 10 for thermally treating a surgical fluid. In the illustrated example, system 10 includes a surgical slush system 12 configured to receive a liquid surgical fluid and to cool the surgical fluid to generate a surgical slush, e.g., for placing organs or other bodily matter on the slush and/or introducing the slush into a body cavity of a patient. In the illustrated example, system 10 also includes an associated surgical warmer system 14 configured to receive a liquid surgical fluid and to heat the surgical fluid to generate a warmed surgical fluid (e.g., for dispensing warmed surgical over a surgical site on or in a patient to irrigate the site and flush away bodily matter. While thermal treatment system 10 is illustrated as being implemented with a combined surgical slush system 12 and surgical warmer system 14, the concepts described herein may be implemented for a surgical slush system that does not include an associated or attached surgical warmer system.

FIG. 1 illustrates thermal treatment system 10 is a freestanding device mounted on wheels movable around a floor surface without the drape positioned over the device. FIG. 2 illustrates a drape 16 positioned over thermal treatment system 10 with a surgical slush present in the basin portion of surgical slush system 12 and a surgical fluid present in the basin portion of surgical warmer system 14. As shown in the example of FIGS. 1 and 2, surgical slush system 12 can include a base 18 and a basin 20. Basin 20 can be supported by and vertically elevated above base 18. Basin 20 may provide an open reservoir into which surgical fluid can be dispensed or other material being processed introduced. Once added to basin 20, the surgical fluid can be temperature adjusted within the basin. For example, basin 20 may be thermally coupled to a thermal treatment device that can cool the surgical fluid placed in the basin to a temperature at or below the freezing point of the surgical fluid. The thermal treatment device thermally coupled to basin 20 can maintain the surgical fluid and/or/generated therefrom at a target temperature.

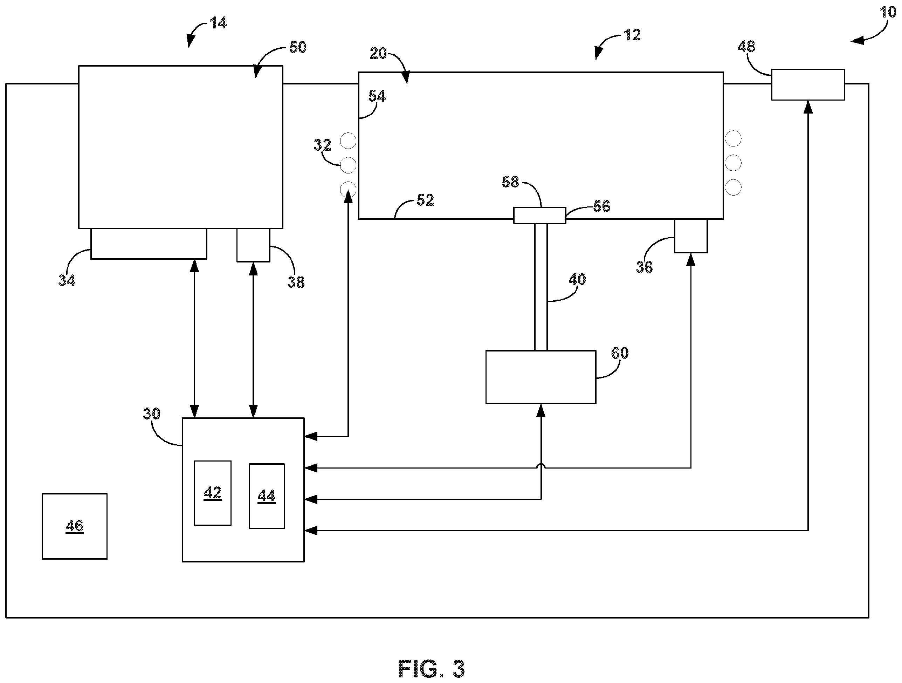

FIG. 3 is a functional block diagram illustrating components of an example configuration of thermal treatment system 10, which includes previously introduced surgical slush system 12 and surgical warmer system 14. System 10 in the illustrated example includes a controller 30, a thermal treatment device 32 configured to cool surgical fluid as part of the surgical slush system 12, a thermal treatment device 34 configured to warm surgical fluid as part of the surgical warmer system 14, and one or more temperature sensors, which are show illustrated as first temperature sensor 36 to measure a temperature associated with the surgical slush basin and a second temperature sensor 38 to measure a temperature associated with the surgical warmer basin. Thermal treatment system 10 in FIG. 3 also includes a piston 40 operable to move relative to basin 20 of surgical slush system 12. In operation, piston 40 can be connected to drape 16 (FIG. 16) and can move relative to basin 20 to dislodge accumulated frozen surgical fluid / slush from the wall surfaces of the drape.

Controller 30 is communicatively coupled to the electronic and controllable components of thermal treatment system 10. Controller 30 can send communication signals to and/or receive communication signals from the various components of thermal treatment system 10 via wired or wireless connections. Controller 30 can include a processor 42 and memory 44. Memory 44 stores software for running controller 30 and may also store data generated or received by processor 42, e.g., from temperature sensors and/or a user interface associated with the device. Processor 42 runs software stored in memory 44 to manage the operation of system 10.

System 10 in FIG. 3 also includes a power source 46 to deliver operating power to the various components of the system. Power source 46 may be a battery that is replaceable or rechargeable. Additionally or alternatively, power source 46 may be a power inlet that receives power from an external source. For example, power source 46 may be a power inlet connected to a cord that plugs into a wall socket to deliver power to system 10. The power received from the external source may recharge a battery contained in system 10 and/or power the various components of the system directly.

During operation, controller 30 can control system 10 with the aid of instructions associated with information stored in memory 44 and with instructions received from an operator via a user interface 48. Instructions executed by controller 30 may, for example, activate piston 40 operate in a reciprocating (e.g., up and down) motion pattern, control thermal treatment device 32 to cool surgical fluid in basin 20 to a target temperature, and/or control thermal treatment device 34 to warm surgical fluid in the basin associated with surgical warmer system 14 to a target temperature. An operator may interact with user interface 48 to start the surgical slush system (e.g., initiate movement of piston 40) and/or to set one or more target temperatures for controlling thermal treatment device 32 and/or 34.

Controller 30 communicates with thermal treatment devices 32, 34 to control the temperature of surgical material placed in each respective basin associated with the thermal treatment device. Thermal treatment device 32 is thermally coupled to basin 20 and operable to adjust the temperature of the basin and any contents therein. Thermal treatment device 32 can be implemented using any device that produces a controllable temperature output. Thermal treatment device 32 can cool basin 20 and any contents therein (e.g., relative to ambient temperature) to produce a semi-frozen slush from surgical fluid placed in the basin. Thermal treatment device 32 may be implemented using a refrigeration unit that typically includes a compressor, a condenser and an expansion control unit connected by appropriate fluid conduits in a closed refrigeration loop of an evaporator. The evaporator may be in the form of a coil wound about the exterior surface of basin 20 in thermal transfer relation therewith. The evaporator can cool the sidewalls and/or bottom wall of basin 20, which may be formed of a thermally conductive material such as stainless steel. In some examples, thermal treatment device 32 cools the contents of basin 20 to a temperature less than -6,7 °C (20°F), such as less than -12,2°C (10°F), less than -17,8°C (0°F), or less than -23,3°C (-10°F).

Thermal treatment device 34 is thermally coupled to a basin 50 of surgical warming system 14 and is operable to adjust the temperature of the basin and any contents therein. Thermal treatment device 34 can be implemented using any device that produces a controllable temperature output. Thermal treatment device 34 can heat basin 50 and any contents therein (e.g., relative to ambient temperature) to generate and/or maintain a warned surgical fluid. Thermal treatment device 34 may be implemented as a warming device that generates heat via electrical resistance. The heat generated by electrical resistance can transfer into basin 50 and any contents therein by conduction, convection, and/or radiation. For example, wiring that generates heat via electrical resistance may be positioned in thermal and/or physical contact with basin 50. Heat generated by thermal treatment device 34 can convey via conduction into basin 50 and any contents therein. In some examples, thermal treatment device 32 is a film heater positioned in thermal communication with basin 50, such as a thin film heater or a thick film heater wrapped at least partially about the basin. Basin 50 can be formed of a thermally conductive material such as stainless steel.

To monitor the temperature of basin 20 and/or basin 50 and/or the contents therein, system 10 may include one or more temperature sensors. In the example of FIG. 3, system 10 includes a first temperature sensor 36 that can sense the temperature of basin 20 and/or the temperature of the contents therein and a second temperature sensor 38 that can sense the temperature of basin 50 and/or the temperature of the contents therein. Each temperature sensor can generate a corresponding signal that is representative of the magnitude of the sensed temperature and communicate the generated signal to controller 30. Controller 30 may receive a signal from each temperature sensor indicative of the temperature measured by the sensor. In various configurations, a temperature sensor may be positioned on an exterior surface of a corresponding basin and can measure the temperature of the contents of the basin through the wall surface and/or the basin may include a port through which temperature sensor extends to measure the temperature of the contents directly in the basin rather than indirectly through a wall surface of the basin.

Surgical slush system 12 includes basin 20. Basin 20 provides a reservoir that receives and holds surgical fluid and/or slush. In general, basin 20 can define any polygonal (e.g., rectangle, square, hexagonal) or arcuate (e.g., circular, elliptical) shape, or even combinations of polygonal and arcuate shapes. In the illustrated examples, basin 20 is shown as a general circular shape and includes a base 52 and one or more sidewalls 54 extending vertically upwardly away from the base. Base 52 and sidewall 54 collectively form a bounded cavity with open top surface that receives and holds the surgical fluid and/or slush. In some examples, sidewall 54 is a sloped instead of a straight sidewall helps prevent surgical fluid and slush from accumulating in corners where the sidewall intersects the base. That being said, in other examples, basin 20 may be formed with straight sidewalls. Further, while basin 20 is illustrated as having an open top surface for adding material to the basin and withdrawing material from the basin, the basin may be closed over its top surface in other configurations.

Basin 20 can include an opening 56 through which piston 40 can extend to connect to drape 16 placed in and/or over the basin. In the illustrated configuration of FIG. 3, opening 56 extends through base 52 of basin 20. For example, opening 56 and piston 40 that extends therethrough may be substantially centered at the bottom of basin 20 with the top surface of the piston configured to attached to the underside of drape 16.

Any type of material may be introduced into and removed from basin 20 and basin 50 during a procedure, including any type of surgical fluid (and/or slush generated therefrom) during a medical procedure. Example types of medical fluid that may be used during a medical procedure include water, saline, or the like. The surgical fluid may or may not include medicament, such as compounds imparting antibacterial properties, anticoagulation / coagulation properties, anesthesia properties, or the like. Alternative materials that may be introduced into basin 20 and/or basin 50 can include tissue or other medical specimens extracted from a patient, blood, platelets, and/or other materials for thermal control or adjustment.

Thermal treatment system 10 may include user interface 48 to allow an operator to interact with system 10 and control different settings. User interface 48 can include a user input through which a clinician inputs information to system 10 and a user output from which the clinician receives information from the system. For example, user interface 48 may include one or more manipulable inputs that the clinician can interact with to adjust settings of system 10. The manipulable user input may be implemented as physically depressible buttons (e.g., switches), portions of a touch screen that a clinician can interact with, or other features that a clinician can interact with to convey information to system 10. The user output of user interface 48 may be a display that provides graphical and/or textual information concerning the operation of system 10. Additionally or alternatively, user interface 48 may include a microphone that detects sounds and/or audible commands from the user and/or an optical detector that detects user action and/or a non-contact user command (e.g., a user gesture).

When surgical slush system 12 is operating (thermal treatment device 32 is cooling), the sterile liquid on drape 16 placed in basin 20 freezes in pieces on the sidewalls of the drape. In order to dislodge these frozen pieces to form sterile slush within the draped basin, piston 40 can actuate relative to basin 20. Movement of piston 40 can flex and/or deform at least a portion of the drape placed over basin 20, causing frozen surgical fluid to dislodge from the wall surfaces of the drape and produce a slush.

Piston 40 can be implemented as a part (e.g., rod, shaft) that moves back and forth relative to basin 20. At least a portion of piston 40 can extend through opening 56 in basin 20 (e.g., through base 52 of basin 50) to connect to and interact with a drape place over the interior surface of basin 20. For example, piston 40 can have a length extending from an end located below basin 20 (e.g., within an interior of surgical slush machine 12) to a terminal end 58 projecting at least partially into the basin (e.g., projecting above the interior surface of base 52 of basin 20). As will be discussed, the terminal end 58 of piston 40 can define a drape engagement member that can engage with a corresponding connection feature on drape 16 to mechanically connect the drape to the piston.

To drive piston 40 during operation of surgical slush system 12, the piston may be connected to a drive source 60. For example, piston 40 may extend vertically downward through opening 56 in base 52 of basin 20 into a drive housing located interiorly of the system. A bearing assembly may be provided about the piston between basin 20 and the drive housing. Drive source 60 may be implemented as a drive motor with a reciprocating shaft that causes piston 40 to move up and down (e.g., vertically with respect to gravity). In other configurations, a drive source 60 such as a drive motor can cause piston 40 to rotate back and forth (in addition to or in lieu of moving up and down) to twist drape 16 to cause accumulated frozen surgical fluid to fall of the drape. Additional details on example configurations for driving piston 40 are described in US Patent No. 5,331,820, granted July 26, 1994, and titled "Method and Apparatus for Forming and Collecting Surgical Slush,".

In use, a drape can be positioned over thermal treatment system 10, including surgical slush system 12, to provide a sterile barrier between the interior surface of basin 20 and/or basin 50 and surgical fluid and/or slush in the basin. The drape can separate the sterile field from a non-sterile field. The drape can be engaged with piston 40 to couple the drape to the piston such that, when the piston moves in response to the control of controller 30, at least a portion of the drape coupled to the piston corresponding moves relative to basin 20.

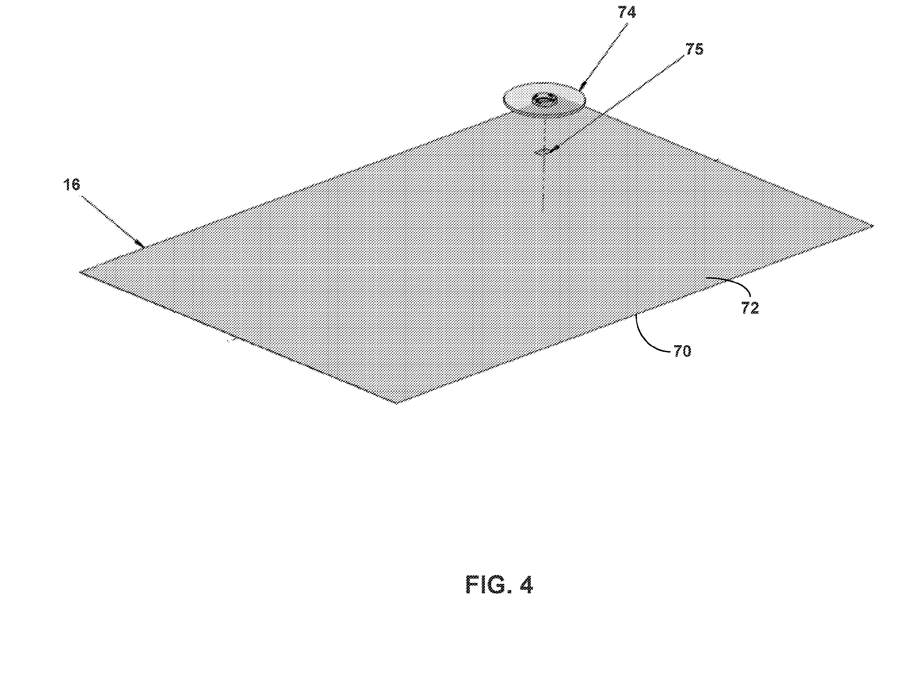

FIG. 4 is a perspective view of one example configuration of drape 16 that can be used on thermal treatment system 10 and/or surgical slush system 12. Drape 16 may be made from a material that is impervious to surgical fluid (and/or slush) and sufficiently flexible to conform to the walls of basin 20 and/or basin 50. The drape can be fitted or non-fitted. A fitted drape can be constructed such that the drape is formed to the contour of basin 20 and/or basin 50 (e.g., matches the size and/or shape of the basin). A non-fitted drape may be a flat or pleated and have a length sufficient to be placed over basin 20 and/or basin 50. In either case, the drape may be placed in basin 20 and/or basin 50 so as to conform to the walls of the basin. In some examples, the drape also extends over the sides of basin 20 and/or basin 50, e.g., hanging down parallel to the base 18 (FIG. 1) of the system. Additionally, in some examples, the drape may have internal partition(s) or divider(s) creating one or more reservoir cavities that are separated from fluid communication with one or more other reservoir cavities. When so configured, the drape can transform a single fluid cavity of basin into multiple fluid cavities.

A disposable drape 16 used with basin 20 and/or basin 50 may have a thickness sufficient to resist tearing and puncturing during normal use but also be sufficiently thin to allow efficient thermal transfer through the drape. While a disposable drape can be made from any suitable materials, in some examples, the drape is made from a polymeric material (e.g., polyethylene, polypropylene, polystyrene, polyurethane). The drape (or a portion thereof) may be transparent or translucent to allow an operator to see features covered by the drape.

Drape 16 can be configured to connect to piston 40 (FIG. 3) of surgical slush system 12. Drape 16 can define a top side 70 configured to face away from basin 20 (when the drape is positioned in and/or over the basin) and a bottom side 72 opposite the top side. Upon placing drape 16 in basin 20, the bottom side 72 of the drape can be configured to face and/or contact the outward facing wall surfaces of the basin (e.g., base 52 and the one or more sidewalls 54).

Drape 16 includes a piston attachment member 74 positioned on the bottom side 72 of the drape. Piston attachment member 74 can be configured (e.g., sized and/or shaped) to engage piston 40 of surgical slush system 12 to connect the drape to the piston. Piston attachment member 74 can been connected to the bottom side 72 of drape 16 in a variety of different ways. In some examples, piston attachment member 74 is adhesively bonded to the drape using an adhesive 75. In other examples, piston attachment member 74 is thermally bonded to the drape (e.g., heat welded to the drape). Other chemical and/or mechanical fixation elements and techniques can be used to fixedly attach piston attachment member 74 to the drape.

Piston attachment member 74 can be positioned at a variety of different locations along drape 16. In some examples, piston attachment member 74 is substantially centered on bottom side 72 of the drape. In some examples, drape 16 is configured to be simultaneously placed over basin 20 of surgical slush system 12 and over basin 50 of surgical warmer system 14. In these examples, drape 16 may define a first drape region configured to be positioned over and/or in contact with the wall surfaces of basin 20 and a second drape region configured to be positioned over and/or in contact with the wall surfaces of basin 50. In these examples, piston attachment member 74 may be substantially centered in the first drape region of the drape configured to be positioned over and/or in contact with the wall surfaces of basin 20, which may be offset from the geometric center of the overall drape.

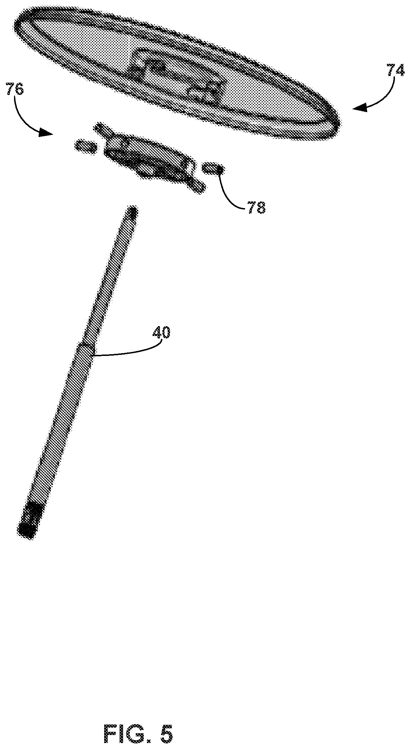

Drape 16 can be connected to piston 40 of surgical slush system 12 by an interconnection defined between piston attachment member 74 carried by the drape and a corresponding drape engagement member on the end of piston 40. FIG. 5 is an exploded perspective view of an example configuration of a connection system that can be used between drape 16 and piston 40 of surgical slush system 12. In the example, piston 40 is shown defining an end 58 that is configured to be positioned in and project into basin 20 (during at least a portion of the range of travel of the piston). The end 58 of piston 40 defines a drape engagement member 76. The drape engagement member 76 is show having one or more alignment members 78 which, in the illustrated example, is provided with four alignment members. Drape engagement member 76 is configured to engage with piston attachment member 74 connected to drape 16 (with the drape to which piston attachment member 74 is connected not being illustrated in FIG. 5).

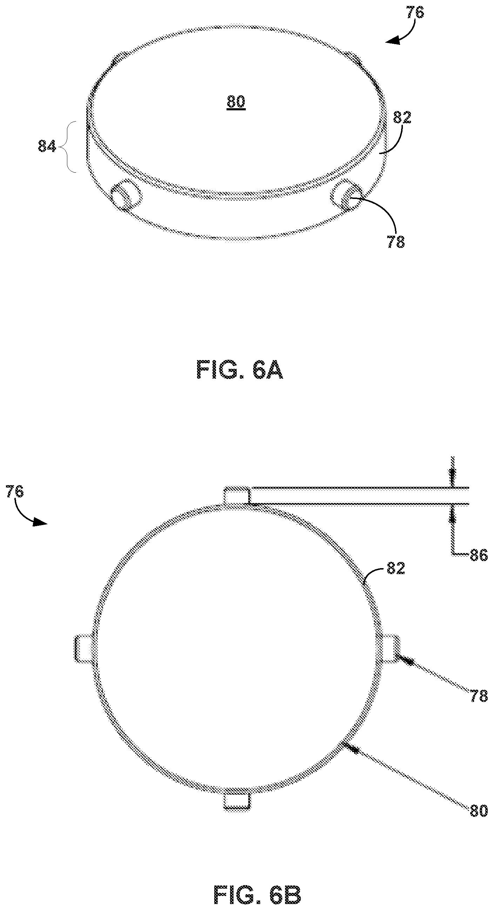

Drape engagement member 76 can have a variety of different configurations. FIGS. 6A and 6B are perspective and top views, respectively, of an example configuration of drape engagement member 76 that can be used on piston 40. As illustrated in this example, drape engagement member 76 can be formed by a plate 80 (referred to as a drape engagement plate) having one or more sidewalls 82. The one or more sidewalls 82 of drape engagement member 76 can define the boundaries of the drape engagement plate 80. One or more alignment members 78 can extend outwardly from the one or more sidewalls 82 defining the boundary or perimeter extent of the drape engagement plate 80.

In general, drape engagement plate 80 can define any polygonal (e.g., rectangle, square, hexagonal) or arcuate (e.g., circular, elliptical) shape, or combinations of polygonal and arcuate shapes. In the illustrated example, drape engagement plate 80 defines a circular cross-sectional shape formed by a single sidewall 82 extending about the perimeter extent of the drape engagement plate. While drape engagement plate 80 of drape engagement member 76 can define a variety of dimensions, in some implementations, the drape engagement plate defines a major length (e.g., diameter) ranging from 25 mm to 100 mm, such as 50 mm to 75 mm. In some examples, the drape engagement plate defines a major length (e.g., diameter) of greater than 50 mm, such as from 53 mm to 55 mm. The thickness 84 of drape engagement plate 80 can also vary. In typical implementations, drape engagement plate 80 may be a comparatively thin structure having a thickness 84 less than the major length (e.g., diameter) of the structure. For example, drape engagement plate 80 may have a thickness 84 within a range from 2 mm to 50 mm, such as from 3 mm to 25 mm, or from 5 mm to 10 mm. In some configurations, drape engagement plate 80 is a solid plate devoid of openings extending through the thickness of the drape engagement plate. In other examples, drape engagement plate 80 may include one or more through openings.

As noted, drape engagement member 76 may include one or more alignment members extending outwardly from drape engagement plate 80. The one or more alignment members 78 may each define a projecting structure that can be engaged by the piston attachment member 74 located on the bottom side of drape 16. In the illustrated configuration, the one or more alignment members are each illustrated as defining a rod having a cylindrical cross-sectional shape extending outwardly from the sidewall 82 of drape engagement plate 80. The one or more alignment members 78 can define any polygonal (e.g., rectangle, square, hexagonal) or arcuate (e.g., circular, elliptical) shape, or combinations of polygonal and arcuate shapes. Further, when configured with multiple alignment members 78 as illustrated in the example of FIGS. 6A and 6B, each alignment member may define the same shape, or at least one alignment member may define a different shape than at least one other alignment member.

The one or more alignment members 78 can extend outwardly from sidewall 82 at a variety of different angles and directions relative to the surface of the side. For example, the one or more alignment member 78 can extend outwardly within the same plane defined by the drape engagement plate 80 or may extend outwardly and an angle (upwardly and/or downwardly) relative to the plane defined by the drape engagement plate. Additionally or alternatively, the one or more alignment members 78 can extend outwardly at an approximately 90° intersecting angle with the sidewall 82 or can extend outwardly at a greater or lesser intersecting angle.

In the illustrated example, each of the one or more alignment members 78 are illustrated as extending radially outwardly from sidewall 82 of drape engagement plate 80. In particular, each alignment member 78 is illustrated as extending outwardly from the sidewall 82 at an approximately 90° intersecting angle with the sidewall within the plane defined by drape engagement plate 80.

Drape engagement member 76 can have any suitable number of alignment members 78. For instance, in different implementations, drape engagement member 76 can have one, two, three, four, five, six, or more alignment members 78. When configured with multiple alignment members 78, the alignment members may be distributed symmetrically about the perimeter of drape engagement plate 80 (e.g., such that there is a substantially equal amount of distance between each pair of adjacent alignment members). Altematively, the alignment members may be distributed asymmetrically about the perimeter of drape engagement plate 80 (e.g., such that there is a different amount of distance between different pairs of adjacent alignment members).

In the illustrated configuration, drape engagement member 76 has four alignment members 78 extending radially outwardly, with the different alignment members being symmetrically positioned about the perimeter of drape engagement plate 80 defined by sidewall 82. As a result, in the illustrated arrangement, each alignment member is positioned approximately 90° apart from each other adjacent alignment member about the perimeter of drape engagement plate 80.

Each alignment member 78 can be implemented with a variety of different dimensions. In some configurations, the one or more alignment members 78 may extend outwardly a distance 86 from the sidewall 82 of drape engagement plate 80 a distance of at least 2 mm, such as at least 5 mm, at least 10 mm, at least 15 mm, or at least 20 mm. The distance 86 the one or more alignment members extend outwardly from the sidewall may be less than 75 mm, such as less than 50 mm, less than 25 mm, less than 20 mm, less than 15 mm, or less than 10 mm. For example, the distance 86 may be within a range from 5 mm to 25 mm, such as from 10 mm to 20 mm. The thickness (e.g., diameter) of each of the one or more alignment members 78 may be within a range from 2 mm to 15 mm, such as from 3 mm to 10 mm, or 4 mm to 6 mm. For example, the one or more alignment member 78 may have a thickness (e.g., diameter) within a range from 4.5 mm to 5 mm, such as approximately 4.75 mm (± 5%). When configured with multiple alignment members 78, each of the one or more alignment members may have the same dimensions, or at least one alignment member may have one or more different dimensions than one or more other alignment members.

Drape engagement member 76 may be permanently affixed to piston 40 (e.g., such that the drape engagement member cannot be separated from the piston without damaging one or both components). Alternatively, drape engagement member 76 may be removably attached to piston 40. As one example, piston 40 may define a threaded end that can be screwed into the bottom side of drape engagement member 76. As another example, an end of piston 40 may be inserted into attachment opening on the bottom side of drape engagement member 76 and a locking or set screw inserted perpendicular to the longitudinal axis of piston 40 to removably attach the piston to the drape engagement member. In these and other configurations, a user may select a particular drape engagement member from a set of two or more different drape engagement members differing from each other in one or more of size and shape (e.g., replacing a current drape engagement member with a different drape engagement member). The user can then engage a drape carrying a piston attachment member 74 corresponding to the selected drape engagement member.

Drape 16 can carry piston attachment member 74 on the bottom side of the drape that is configured (e.g., sized and shaped) to connect to the drape engagement member 76 located on the end of piston 40 of surgical slush machine 12. The specific configuration of piston attachment member 74 may vary depending on the configuration of the corresponding drape engagement member 76. In general, piston attachment member 74 may define a body having an opening into which drape engagement member 76 is configured to be inserted.

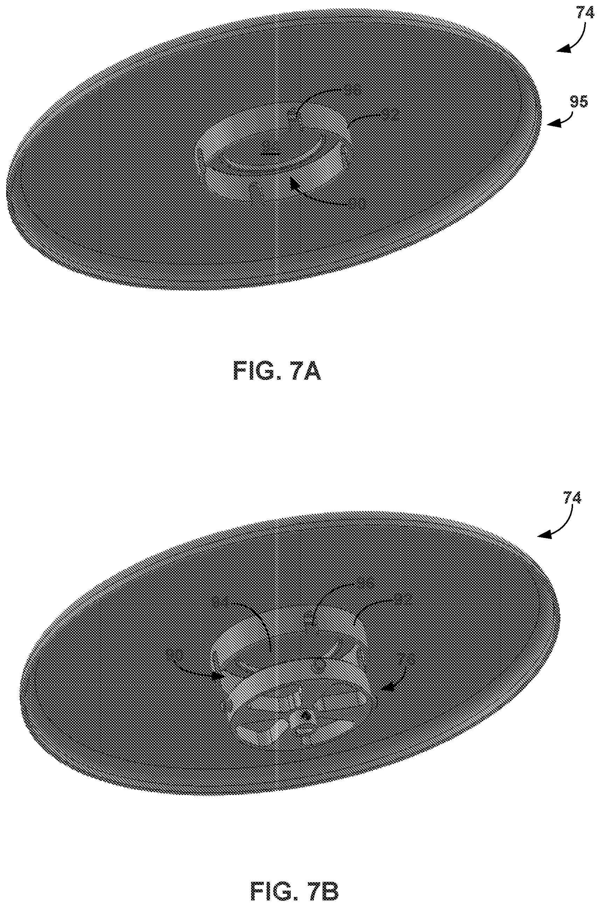

FIGS. 7A and 7B (collectively referred to as "FIG. 7") are different perspective views illustrating a bottom side of an example piston attachment member 74. FIG. 7A illustrates piston attachment member 74 separated from drape engagement member 76 associated with piston 40. FIG. 7B illustrates drape engagement member 76 offset from an insertable into the example configuration of piston attachment member 74 illustrated in FIG. 7A. In both FIG. 7A and 7B, the top side of the example piston attachment member 74 would be connected to a drape as described herein, although such example drape is not illustrated for purposes of discussion.

As shown in the example of FIG. 7, piston attachment member 74 may define a receiving cavity 90 configured to receive at least a portion of drape engagement member 76. For example, receiving cavity 90 may be an opening or void space defined by one or more wall surfaces of piston attachment member 74 that is configured to receive drape engagement plate 80 and the one or more alignment members 78 extending outwardly from the drape engagement plate. Receiving cavity 90 of piston attachment member 74 can be positioned over the drape engagement plate and one or more alignment members. To attach piston attachment member 74 to drape engagement member 76, for example, receiving cavity 90 of the piston attachment member can be positioned over drape engagement member 76 (including drape engagement plate 80 and the one or more alignment member 78) and pressed onto the drape engagement member. Piston attachment member 74 can be pressed vertically downwardly on top of drape engagement member 76 (and/or sidewise on the drape engagement member depending on the configuration) and, in some examples, rotated relative to the drape engagement member.

Receiving cavity 90 can be defined by number of structural configurations that form an opening configured to receive at least a portion of drape engagement member 76. In some examples, including the illustrated example of FIG. 7, piston attachment member 74 includes one or more downwardly extending sidewalls 92 that defined a perimeter extent of receiving cavity 90. For example, piston attachment member 74 may include a base wall 94 that bounds the deepest extent of receiving cavity 90 and one or more wall surfaces 92 that extend outwardly from base wall 94 to define the thickness or depth of the receiving cavity.

As illustrated, piston attachment member 74 defines a plate 95 that includes receiving cavity 90 (including sidewall 92 and base wall 94 extending therefrom) substantially centered on plate. Plate 95 can define a region of increased surface area compared to the cross-sectional area of receiving cavity 90 and/or drape engagement member 76, allowing force transferred by piston 40 to be distributed over a larger region of drape 16 via plate 95. When utilizing a structure of a larger surface area such as plate 95 attached to drape 16, the entire cross-sectional area of the plate may be at least 50% bigger than the cross-sectional area of receiving cavity 90, such as at least 100% bigger, at least 150% bigger, or at least 200% bigger.

Receiving cavity 90 of piston attachment member 74 can define any shape, and may typically have a cross-sectional shape complementary to (e.g., the same as) the shape of drape engagement plate 80. In the illustrated example, piston attachment member 74 defines a circular cross-sectional shape formed by sidewall 92 extending about the perimeter defining the receiving cavity. Receiving cavity 90 can define other polygonal (e.g., rectangle, square, hexagonal) or arcuate (e.g., circular, elliptical) shape, or combinations of polygonal and arcuate shapes.

The dimensions of receiving cavity 90 can vary depending, for example, on the size of drape engagement plate 80 of drape engagement member 76. Receiving cavity 90 may have a size substantially the same as the size of drape engagement plate 80 (e.g., ± 5%). For instance, in some examples, receiving cavity 90 has a cross-sectional size the same as or slightly larger than the size of drape engagement plate 80. This can allow receiving cavity 90 to be positioned over drape engagement plate 80 without bending the sidewall or enlarging the size of the receiving cavity to fitted over the engagement plate. In other examples, receiving cavity 90 has a cross-sectional size slightly larger than the size of drape engagement plate 80. For example, receiving cavity 90 may have a cross-sectional size (e.g., diameter) that is between 0.1% and 10% smaller than the cross-sectional size of drape engagement plate 80, such as between 0.2% and 5% smaller, or between 1% and 5% smaller. Receiving cavity 90 may have the same cross-sectional size across the entire thickness of the receiving cavity, or one or more portions may define a region of different cross-sectional size than one or other regions. For example, receiving cavity 90 may have a cross-sectional size substantially the same as or slightly larger than drape engagement plate 80 extending from base 94 downwardly along sidewall 92 and then include another region adjacent the terminal end of sidewall 92 defining a region slightly smaller than drape engagement plate 80.

In various examples, piston attachment member 74 (the entirety of the member or at least a portion thereof) may be formed of a pliable polymeric material that can bend and deform (e.g., stretch) to allow the receiving cavity to be placed over the drape engagement plate. In different implementations, piston attachment member 74 may be formed of polyethylene, polypropylene, polystyrene, polyurethane, and/or other polymeric materials. In various implementations, drape engagement member 76 may also be formed of a polymeric material (including any of the aforementioned polymeric materials), a metal (e.g., stainless steel), and/or other material.

In some examples, receiving cavity 90 of piston attachment member 74 defines a salad sidewall surface extending about the entire perimeter of the receiving cavity without any openings or cutouts extending through the sidewall surface. In these implementations, sidewall 92 may be formed of a sufficiently deformable and/or pliable material so as to allow the sidewall to be deformed and pulled over the one or more alignment members 78 of drape engagement member 76 (e.g., with the sidewall then biasing back toward in natural position to retain the piston attachment member to the drape engagement). In other examples, receiving cavity 90 defines one or more cutouts configured to accommodate the one or more alignment member 78 of drape engagement member 76.

For example, with reference to FIG. 7, piston attachment member 74 includes one or more cutouts 96. The one or more cutouts 96 can define openings extending through the thickness of sidewall 92. The one or more cutouts are configured (e.g., sized, shaped, and/or positioned) to receive the one or more alignment members 78 defined by drape engagement member 76. The number and positioning of the one or more cutouts 96 defined by piston attachment member 74 may correspond to the number and positioning of the one or more alignment members 78, as discussed above.

For instance, in different implementations, piston attachment member 74 can have one, two, three, four, five, six, or more cutouts 96. When configured with multiple cutouts 96, the cutouts may be distributed symmetrically about the perimeter of receiving cavity 90 (e.g., such that there is a substantially equal amount of distance between each pair of adjacent cutouts). Altematively, the cutouts may be distributed asymmetrically about the perimeter of receiving cavity 90 (e.g., such that there is a different amount of distance between different pairs of adjacent cutouts).

In the illustrated configuration, piston attachment member 74 has four cutouts 96 extending through the thickness of sidewall 92, with the different cutouts being symmetrically positioned about the perimeter of receiving cavity 90 defined by the sidewall. As a result, in the illustrated arrangement, each cutout is positioned approximately 90° apart from each other adjacent cutout about the perimeter of the one or more sidewalls defining receiving cavity 90.

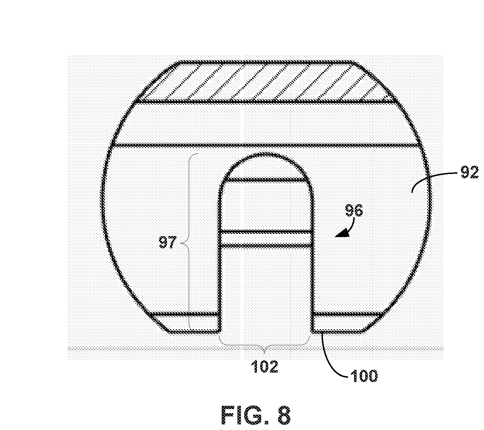

FIG. 8 is an expanded view of an example cutout configuration that can be used for the one or more cutouts 96. As shown in this example, cutout 96 may be or include a vertically extending portion 98 extending parallel to the length of sidewall 92. The vertically extending portion 98 can extend upwardly from a terminal end 100 of the one or more downwardly extending sidewalls 92. Cutout 96 can define a width 102 extending across the cutout (e.g., perpendicular to the length of the cutout). In some examples, the width 102 of cutout 96 is sized larger than the cross-sectional size (e.g., diameter, width) of the alignment member 78 to be inserted into and received by the cutout. When so configured, alignment member 78 can be positioned in cutout 96 without coupling the alignment member to the cutout. For example, alignment member 78 can be positioned in cutout 96 with sections of sidewall 92 divided by the cutout positioned on either side of the alignment member. This can function to align the sidewall sections relative to alignment member 78 (and/or prevent and/or inhibit rotation there between) without forming a mechanical connection between the alignment member and a portion of piston attachment member 96.

In some examples, the width 102 of cutout 96 is sized substantially the same as or smaller than the cross-sectional size (e.g., diameter, width) of the alignment member 78 to be inserted into and received by the cutout. For example, cutout 96 may have a width 102 that is between 0.1% and 10% smaller than the cross-sectional size of alignment member 78, such as between 0.2% and 5% smaller, or between 1% and 5% smaller. The width 102 may be the same across the entire length 97 of the cutout, or one or more portions may define a region of different width than one or more other regions.

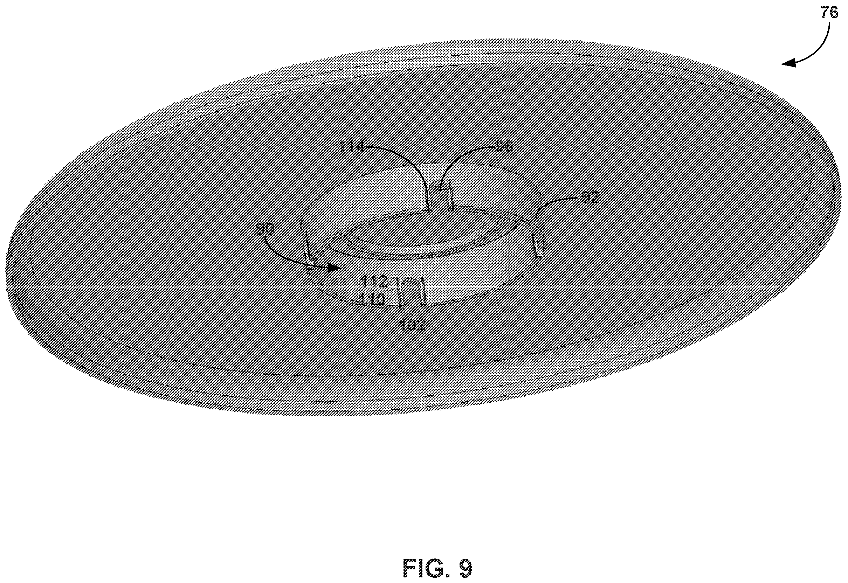

When configured with one or more cutouts 96, the one or more cutouts can have a variety of sizes and configurations. FIG. 9 is a perspective view illustrating a bottom side of an example configuration of piston attachment member 74 where like elements refer to like features discussed above. In particular, FIG. 9 illustrates an example configuration of piston attachment member 74 where the width 102 of cutout 96 is sized smaller than the cross-sectional size (e.g., diameter, width) of the alignment member 78 to be inserted into and received by the cutout.

For example, cutout 96 can include a first region 110 having a width 102 that is smaller than the cross-sectional size of alignment member 78 and a second region 112 having a width 102 that is the same as or larger than the cross-sectional size alignment member. In the illustrated arrangement, the first region 110 extends from the terminal end 100 of sidewall 92 upwardly along the length of cutout 96. The second region 112 is positioned closer to the bottom surface bounding the receiving cavity 90 offset from the terminal end 100 of sidewall 92 than the first region. In some examples, a slit 114 may extend parallel to the length of cutout 96 on one or both sides of the cut. In use, an operator can push piston attachment member 74 over drape engagement member 76, causing the one or more alignment members 78 to enter the corresponding one or more cutouts 96. As the one or more alignment members enter the first region 110 of cutout 96, the comparatively large size of the alignment member relative to the width 102 of the cutout can cause the sidewalls bounding the cutout to push laterally away. The one or more slits 114 can help facilitate this lateral deformation of the bounding sidewalls. In either case, alignment member 78 can continue advancing along the vertical length of cutout 96 until entering second region 112. The comparatively larger size of second region 112 may allow the sidewalls bounding cutout 96 to return to their natural position, position at least a portion of the sidewalls defining the first region 110 under the alignment member and helping to retain the alignment member in the cutout. In this way, the alignment member 78 can be press fit into the cutout 96, with the sidewalls bounding the cutout helping to trap the alignment member and the cutout.

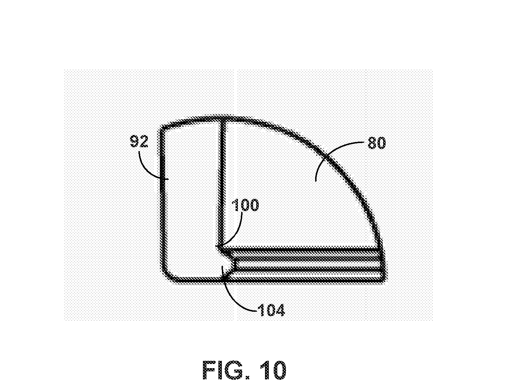

Independent of the number, size, and configuration of the one or more cutouts 96, piston attachment member 74 may be configured to wrap at least partially about a bottom side of drape engagement member 76 to help form a mechanical connection between the two components. FIG. 10 is an expanded view of a portion of an example configuration piston attachment member 74. As shown in this example, the piston attachment member includes a lateral wall 104 extending inwardly from the terminal end 100 of the downwardly extending sidewall 92. The lateral wall 104 can extend at least partially under a bottom side of the drape engagement plate 80 to help retain the piston attachment member to the drape engagement member.

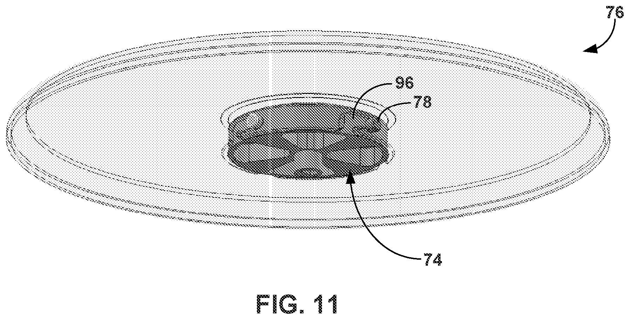

FIG. 11 is a perspective view illustrating a bottom side of another example configuration of piston attachment member 74 where like elements refer to like features discussed above. In particular, FIG. 11 illustrates an example configuration of piston attachment member 74 having one or more cutouts 96 configured to rotationally interlock with the one or more alignment members 78. During use, piston attachment member 74 carried by drape 16 may be pressed vertically downwardly on top of piston attachment member 74 and subsequently rotated within a particular vertical plane to interlock the piston attachment member to the drape engagement. FIG. 11 illustrates piston attachment member 74 inserted into and rotationally interlocked with drape engagement member 76.

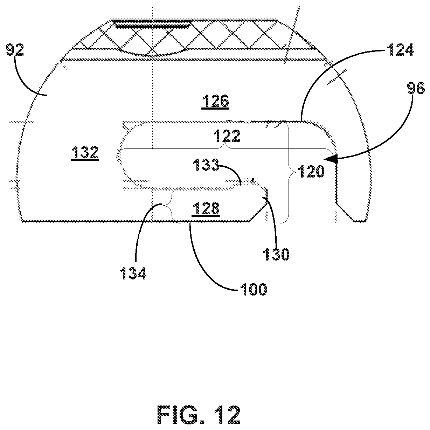

FIG. 12 is an expanded view of the cutout configuration of the example piston attachment member 74 shown in FIG. 11. As shown in this example, cutout 96 includes a vertical portion 120 extending upwardly from the terminal end 100 of the downwardly extending sidewall 92. Cutout 96 also includes a lateral portion 122 extending laterally at an angle from the vertical portion 120. In particular, lateral portion 122 is illustrated as extending laterally at a 90 degree angle, although may extend at a different angle (e.g., an angle less than 90 degrees) in other configurations. During assembly, a user can press piston attachment member 74 on drape engagement member 76, in the process inserting one or more alignment members 78 into the vertical portion 120 of the one or more cutouts 96. The user can press the cutout 96 down until a top wall surface 124 bounding the vertical extent of vertical portion 120 is contacted by the alignment member 78.

With the alignment member 78 positioned in the vertical portion 120 of cutout 96, the user can rotate piston attachment member 74 relative to drape engagement member 76, causing the alignment member 78 positioned in the vertical portion of the cutout to move into the lateral portion 122 of the cutout. The extent of rotation may be dictated, for example, by the size and configuration of cutout 96 (e.g., the length of the lateral portion 122 of the cutout) in the size and configuration of alignment member 78. In various examples, the user may rotate piston attachment member 74 relative to drape engagement member 76 an amount that is within a range from 1° to 45°, such as from 5° to 25°, or from 10° to 20°.

The cutout 96 configured with a laterally extending portion 122 can have a variety of configurations. In some examples, the lateral portion 122 is bounded on a topside by base wall 94 the bounds the deepest extent of receiving cavity 90. The lower portion of the cutout defining lateral portion 122 can be formed by laterally extending section of sidewall. In the illustrated example, the lateral portion 122 of cutout 96 divides the downwardly extending sidewall 92 into an upper sidewall portion 126 and a lower sidewall portion 128 which bound the lateral portion of the cutout between the two sidewall portions. In particular, the lower sidewall portion 128 extends from a terminal end 130 bounded by the vertical portion 120 of the cutout to an undivided portion 132 of the downwardly extending sidewall 92. In some examples, including that illustrated in FIG. 12, the terminal end 130 of the lower sidewall portion 128 defines an upwardly extending retention lip 133. Retention lip 133 may be a hump or enlargement that the alignment member 78 fits behind, after being rotationally interlocked, to help prevent inadvertent movement of the alignment member vote of the lateral portion 122 of the cutout and subsequently the vertical portion 120 of the cutout.

The size and configuration of features defining cutout 96 in the example of FIG. 12 may vary. In some examples, the lower sidewall portion 128 defines a length parallel to the lateral portion 122 of the cutout and a width 134 parallel to the vertical portion 120 of the cutout. The width 134 of the lower sidewall portion 128 may be controlled to control the breakout force required to separate piston attachment member 74 (and the drape attached thereto) from drape engagement member 76 (and the piston attached thereto). For example, the width 134 of the lower sidewall portion 128 may be sufficiently large that the lower sidewall portion retains an alignment member 78 inserted into lateral portion 122 during normal operation of the surgical slush machine. That is, lower sidewall portion 128 can withstand the up-and-down reciprocating forces generated by piston 40 without deforming or breaking and causing alignment member 78 to disengage from the cutout retaining the alignment member.

However, in some implementations, the width 134 of the lower sidewall portion 128 may be sufficiently small that the alignment member 78 can pull through the lower sidewall portion if the force generated between piston attachment member 74 and drape engagement member 76 exceeds a threshold (e.g., a threshold corresponding to damage of the surgical slush machine). For example, in instances where ice or other matter has built up on drape 16 and is limiting piston 40 from retracting to its lowest most position associated with normal operation, lower sidewall portion 128 may be configured to allow alignment member 78 to pull through the sidewall portion and thereby disengage the piston from the drape. In various implementations, lower sidewall portion 128 may be configured to break away (e.g., by providing a fracture line or other weakened region configured to break upon exceeding a force threshold) and/or configured to elastically or inelastically deform in response to exceeding a force threshold that allows the alignment member 78 to pull through the lower sidewall portion 128 and disengage.

In some examples, the width 134 of the lower sidewall portion 128 is less than 10 mm, such as less than 5 mm, or less than 3 mm. For example, the width 134 of the lower sidewall portion 128 may be within a range from 1 mm to 5 mm, such as from 2 mm to 4 mm.

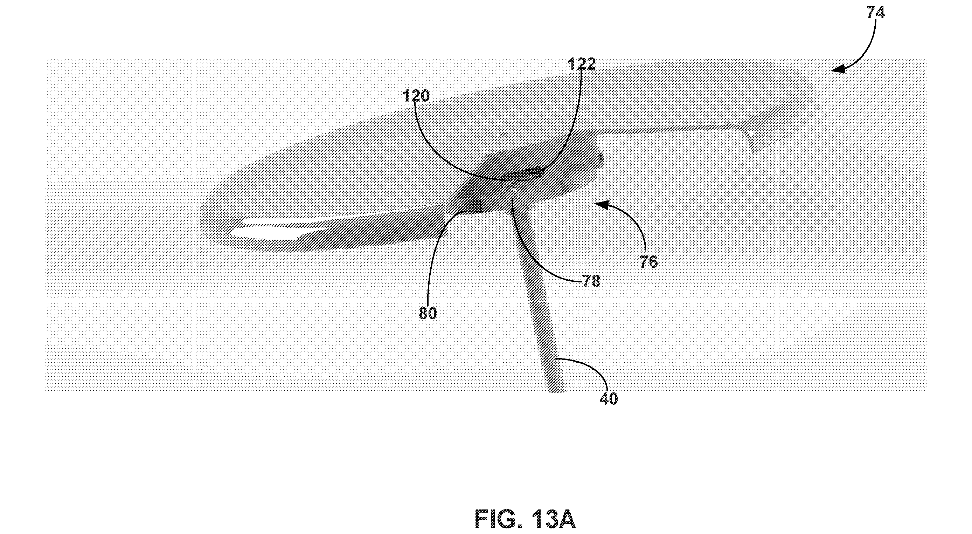

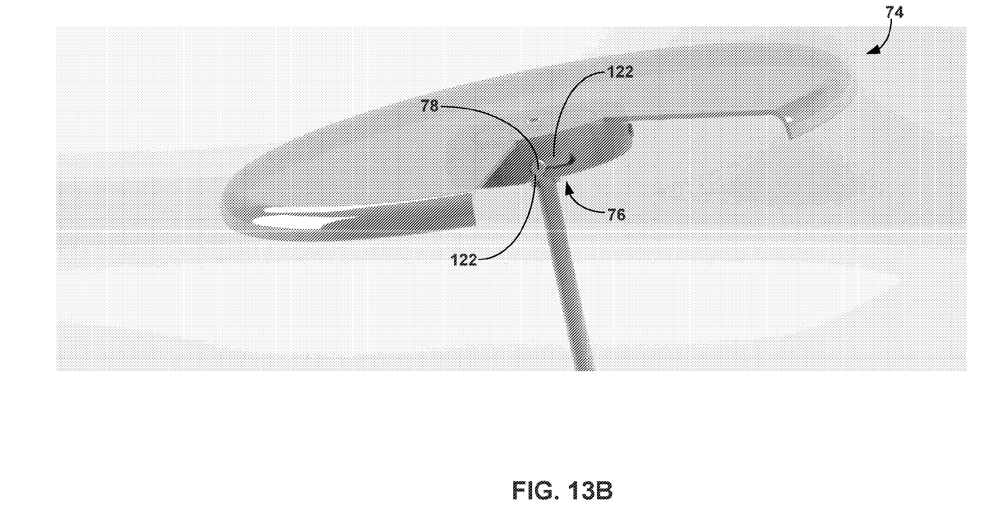

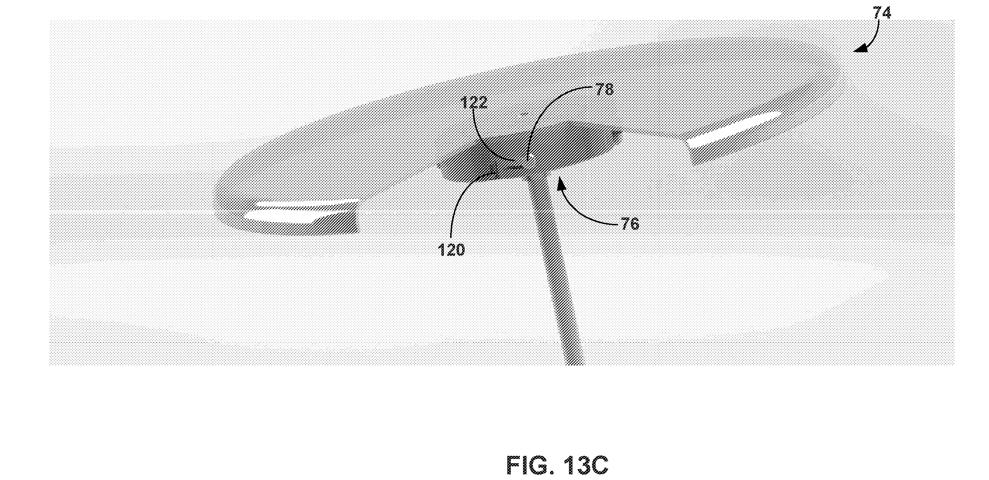

FIGS. 13A-13C illustrate assembly of an example piston attachment member 74 according to FIG. 12 to an example drape engagement member 76. As shown in these figures, a user can press piston attachment member 74 on drape engagement member 76, in the process inserting one or more alignment members 78 into the vertical portion 120 of each of the one or more cutouts 96. The process of press piston attachment member 74 on drape engagement member 76 can insert drape engagement plate 80 into receiving cavity 90. With the alignment member 78 positioned in the vertical portion 120 of cutout 96, the user can rotate piston attachment member 74 relative to drape engagement member 76, causing the alignment member 78 positioned in the vertical portion of the cutout to move into the lateral portion 122 of the cutout. This can help interlock the piston attachment member 74 to the drape engagement member 76.

It should be appreciated that the descriptive terms "top" and "bottom" with respect to the configuration and orientation of components described herein are used for purposes of illustration based on the orientation in the figures. The arrangement of components in real world application may vary depending on their orientation with respect to gravity. Accordingly, unless otherwise specified, the general terms "first" and "second" may be used interchangeably with the terms "top" and "bottom" without departing from the scope of disclosure.

Thermal treatment systems, including surgical slush systems, as described in this disclosure may be implemented, at least in part, in hardware, software, firmware or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a non-transitory computer-readable medium, such as a computer-readable storage medium, containing instructions. Instructions embedded or encoded in a computer-readable storage medium may cause a programmable processor, or other processor, to perform the method, e.g., when the instructions are executed. Non-transitory computer readable storage media may include volatile and/or non-volatile memory forms including, e.g., random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various examples have been described. These and other examples are within the scope of the following claims.

## Claims

1. A drape for a surgical slush system, the drape comprising:
a basin portion configured to be inserted into a basin of a surgical slush system, the basin portion comprising a top side (70) configured to face away from the basin of the surgical slush system and a bottom side (72) configure to face the basin of the surgical slush system;
a flexible side sheet extending about the basin portion and configured be positioned draping side portions of the surgical slush system; and
a piston attachment member (74) on the bottom side of the basin portion, the piston attachment member comprising at least one downwardly extending sidewall (92) defining a perimeter of a receiving cavity (90) and at least one cutout (96) extending through the downwardly extending sidewall, **characterised in that** the at least one cutout is configured to receive an alignment member of the surgical slush system;
and **in that** the at least one cutout (96) comprises a vertical portion (120) extending upwardly from a terminal end (100) of the at least one downwardly extending sidewall (92) and a lateral portion (122) extending laterally at an angle from the vertical portion.

2. The drape of claim 1, wherein the lateral portion (122) of the at least one cutout (96) divides the at least one downwardly extending sidewall (92) into an upper sidewall portion (126) and a lower sidewall portion (128) bounding the lateral portion of the at least one cutout.

3. The drape of claim 2, wherein the lower sidewall portion (122) extends from a terminal end (100) bounded by the vertical portion (120) of the least one cutout to an undivided portion (132) of the at least one downwardly extending sidewall (92), and the terminal end (130) of the lower sidewall portion defines a retention lip (133).

4. The drape of either of claims 2 or 3, wherein the lower sidewall portion (128) defines a length parallel to the lateral portion (122) of the at least one cutout (92) and a width parallel to the vertical portion (12) of the least one cutout, and the width of the lower sidewall portion is less than 5 mm.

5. The drape of any one of claims 1-4, wherein the piston attachment member further comprises a lateral wall (104) extending inwardly from a terminal end (100) of the at least one downwardly extending sidewall (92).

6. The drape of any one of claims 1-5, wherein the at least one cutout (92) has a length and a width, and the width of the at least one cutout is smaller along at least a portion of the length than a width of the at least one alignment member, thereby configuring the at least one cutout to be press fit onto the at least one alignment member.

7. The drape of any one of claims 1-6, wherein:
the receiving cavity (90) defines a circular shape; and
the at least one cutout (96) is four cutouts positioned approximately 90 degrees apart from each other about a perimeter of the receiving cavity.

8. The drape of claim 2, wherein the lower sidewall portion (128) defines a length parallel to the lateral portion (122) of the cutout and a width (134) parallel to the vertical portion (120) of the cutout.

9. The drape of claim 8, wherein a width (134) of the lower sidewall portion (128) determines a breakout force required to separate the piston attachment member from a drape engagement member of the surgical slush system.

10. The drape of claim 9, wherein the width (134) of the lower sidewall portion (128) is such that the lower sidewall portion retains the alignment member inserted into the lateral portion (122) during normal operation of the surgical slush machine.

11. The drape of claim 10, wherein the width (134) of the lower sidewall portion (128) is such that the alignment member pulls through the lower sidewall portion if the force generated between the piston attachment member and the drape engagement member exceeds a threshold.

12. The drape of claim 11, wherein threshold corresponds to a force sufficient to damage the surgical slush machine.

13. The drape of claims 11 or 12, wherein the lower sidewall portion (128) is configured to break away if the threshold is reached.

14. The drape of claim 13, wherein the lower sidewall portion (128) comprises a weakened region configured to break upon exceeding the threshold.

15. The drape of claims 11 or 12, wherein the lower sidewall portion (128) is configured to deform if the threshold is reached.

## Patentansprüche

1. Drape für ein chirurgisches Slush-System, das Drape umfassend:
einen Beckenabschnitt, der so konfiguriert ist, dass er in ein Becken eines chirurgischen Slush-Systems eingesetzt werden kann, wobei der Beckenabschnitt eine Oberseite (70) umfasst, die so konfiguriert ist, dass sie von dem Becken des chirurgischen Slush-Systems weg zeigt, und eine Unterseite (72) umfasst, die so konfiguriert ist, dass sie zum Becken des chirurgischen Slush-Systems hinzeigt;
eine flexible Seitenfolie, die sich um den Beckenabschnitt erstreckt und so konfiguriert ist, dass sie die Seitenabschnitte des chirurgischen Slush-Systems einhüllt; und
ein Kolbenbefestigungselement (74) an der Unterseite des Beckenabschnitts, wobei das Kolbenbefestigungselement mindestens eine sich nach unten erstreckende Seitenwand (92) umfasst, die einen Umfang eines Aufnahmeraums (90) definiert, und mindestens eine Aussparung (96) umfasst, die sich durch die nach unten verlaufende Seitenwand erstreckt, **dadurch gekennzeichnet, dass** die mindestens eine Aussparung so konfiguriert ist, dass sie ein Ausrichtungselement des chirurgischen Slush-Systems aufnehmen kann;
und dadurch, dass die mindestens eine Aussparung (96) einen vertikalen Abschnitt (120) umfasst, der sich von einem Endpunkt (100) der mindestens einen nach unten verlaufenden Seitenwand (92) nach oben erstreckt, und einen seitlichen Abschnitt (122), der sich seitlich in einem Winkel von dem vertikalen Abschnitt erstreckt.

2. Drape nach Anspruch 1, wobei der seitliche Abschnitt (122) der mindestens einen Aussparung (96) die mindestens eine nach unten verlaufende Seitenwand (92) in einen oberen Seitenwandabschnitt (126) und einen unteren Seitenwandabschnitt (128) teilt, die den seitlichen Abschnitt der mindestens einen Aussparung begrenzen.

3. Drape nach Anspruch 2, wobei der untere Seitenwandabschnitt (122) von einem Endpunkt (100), der durch den vertikalen Abschnitt (120) der mindestens einen Aussparung begrenzt ist, zu einem ungeteilten Abschnitt (132) der mindestens einen sich nach unten erstreckenden Seitenwand (92) verläuft und der Endpunkt (130) des unteren Seitenwandabschnitts eine Haltekante (133) definiert.

4. Drape entweder nach Anspruch 2 oder 3, wobei der untere Seitenwandabschnitt (128) eine Länge parallel zu dem seitlichen Abschnitt (122) der mindestens einen Aussparung (92) und eine Breite parallel zu dem vertikalen Abschnitt (12) der mindestens einen Aussparung definiert, und wobei die Breite des unteren Seitenwandabschnitts weniger als 5 mm beträgt.

5. Drape nach einem der Ansprüche 1 bis 4, wobei das Kolbenbefestigungselement ferner eine Seitenwand (104) umfasst, die sich von einem Endpunkt (100) der mindestens einen nach unten verlaufenden Seitenwand (92) erstreckt.

6. Drape nach einem der Ansprüche 1 bis 5, wobei die mindestens eine Aussparung (92) eine Länge und eine Breite aufweist, und die Breite der mindestens einen Aussparung entlang mindestens eines Abschnitts der Länge geringer ist als eine Breite des mindestens einen Ausrichtungselements, wodurch die mindestens eine Aussparung so konfiguriert ist, dass sie auf das mindestens eine Ausrichtungselement aufgepresst wird.

7. Drape nach einem der Ansprüche 1 bis 6, wobei:
der Aufnahmeraum (90) eine Kreisform definiert; und
die mindestens eine Aussparung (96) aus vier Aussparungen besteht, die jeweils ungefähr 90 Grad voneinander um einen Umfang des Aufnahmeraums herum positioniert sind.

8. Drape nach Anspruch 2, wobei der untere Seitenwandabschnitt (128) eine Länge parallel zu dem seitlichen Abschnitt (122) der Aussparung und eine Breite (134) parallel zu dem vertikalen Abschnitt (120) der Aussparung definiert.

9. Drape nach Anspruch 8, wobei eine Breite (134) des unteren Seitenwandabschnitts (128) eine Ausbrechkraft bestimmt, die erforderlich ist, um das Kolbenbefestigungselement von einem Drape-Eingriffselement des chirurgischen Slush-Systems zu trennen.

10. Drape nach Anspruch 9, wobei die Breite (134) des unteren Seitenwandabschnitts (128) so ist, dass der untere Seitenwandabschnitt das in den seitlichen Abschnitt (122) eingesetzte Ausrichtungselement während eines normalen Betriebs der chirurgischen Slush-Maschine hält.

11. Drape nach Anspruch 10, wobei die Breite (134) des unteren Seitenwandabschnitts (128) so ist, dass das Ausrichtungselement durch den unteren Seitenwandabschnitt zieht, wenn die zwischen dem Kolbenbefestigungselement und dem Drape-Ausrichtungselement erzeugte Kraft einen Schwellenwert überschreitet.

12. Drape nach Anspruch 11, wobei ein Schwellenwert einer Kraft entspricht, die ausreicht, um die chirurgische Slush-Maschine zu beschädigen.

13. Drape nach Anspruch 11 oder 12, wobei der untere Seitenwandabschnitt (128) so konfiguriert ist, dass er ausbricht, wenn der Schwellenwert überschritten wird.

14. Drape nach Anspruch 13, wobei der untere Seitenwandabschnitt (128) einen verdünnten Bereich umfasst, der so konfiguriert ist, dass er bei Überschreiten des Schwellenwerts ausbricht.

15. Drape nach Anspruch 11 oder 12, wobei der untere Seitenwandabschnitt (128) so konfiguriert ist, dass er sich verformt, wenn der Schwellenwert erreicht wird.

## Revendications

1. Champ opératoire destiné à un système de glace chirurgicale, le champ opératoire comprenant :
une partie bassin conçue pour être insérée dans un bassin d'un système de glace chirurgicale, la partie bassin comprenant un côté supérieur (70) conçu pour faire face au bassin du système de glace chirurgicale et un côté inférieur (72) conçu pour faire face au bassin du système de glace chirurgicale ;
une feuille latérale flexible s'étendant autour de la partie bassin et conçue pour être positionnée de manière à recouvrir les parties latérales du système de glace chirurgicale ; et
un élément de fixation de piston (74) sur le côté inférieur de la partie bassin, l'élément de fixation de piston comprenant au moins une paroi latérale s'étendant vers le bas (92) définissant un périmètre d'une cavité de réception (90) et au moins une découpe (96) s'étendant à travers la paroi latérale s'étendant vers le bas, **caractérisé en ce que** ladite au moins une découpe est conçue pour recevoir un élément d'alignement du système de glace chirurgicale ;
et **en ce que** ladite au moins une découpe (96) comprend une partie verticale (120) s'étendant vers le haut à partir d'une extrémité terminale (100) de ladite au moins une paroi latérale s'étendant vers le bas (92) et une partie latérale (122) s'étendant latéralement à un certain angle à partir de la partie verticale.

2. Champ opératoire selon la revendication 1, dans lequel ladite au moins partie latérale (122) de ladite au moins une découpe (96) divise ladite au moins une paroi latérale s'étendant vers le bas (92) en une partie de paroi latérale supérieure (126) et une partie de paroi latérale inférieure (128) délimitant ladite au moins partie latérale de ladite au moins une découpe.

3. Champ opératoire selon la revendication 2, dans lequel la partie de paroi latérale inférieure (122) s'étend depuis une extrémité terminale (100) délimitée par la partie verticale (120) de ladite au moins une découpe jusqu'à une partie non divisée (132) de ladite au moins une paroi latérale s'étendant vers le bas (92), et l'extrémité terminale (130) de la partie de paroi latérale inférieure définit une lèvre de retenue (133).

4. Champ opératoire selon l'une quelconque des revendications 2 ou 3, dans lequel la partie de paroi latérale inférieure (128) définit une longueur parallèle à la partie latérale (122) de ladite au moins une découpe (92) et une largeur parallèle à la partie verticale (12) de ladite au moins une découpe, et la largeur de la partie de paroi latérale inférieure est inférieure à 5 mm.

5. Champ opératoire selon l'une quelconque des revendications 1 à 4, dans lequel l'élément de fixation de piston comprend en outre une paroi latérale (104) s'étendant vers l'intérieur à partir d'une extrémité terminale (100) de ladite au moins une paroi latérale s'étendant vers le bas (92).

6. Champ opératoire selon l'une quelconque des revendications 1 à 5, dans lequel ladite au moins une découpe (92) présente une longueur et une largeur, et la largeur de ladite au moins une découpe est plus petite le long de ladite au moins une partie de la longueur qu'une largeur dudit au moins un élément d'alignement, de qui permet de concevoir ladite au moins une découpe pour être ajustée par pression sur ledit au moins un élément d'alignement.

7. Champ opératoire selon l'une quelconque des revendications 1 à 6, dans lequel :
la cavité de réception (90) définit une forme circulaire ; et
ladite au moins une découpe (96) correspond à quatre découpes disposées approximativement à 90 degrés les unes des autres autour d'un périmètre de la cavité de réception.

8. Champ opératoire selon la revendication 2, dans lequel la partie de paroi latérale inférieure (128) définit une longueur parallèle à la partie latérale (122) de la découpe et une largeur (134) parallèle à la partie verticale (120) de la découpe.

9. Champ opératoire selon la revendication 8, dans lequel une largeur (134) de la partie de paroi latérale inférieure (128) détermine une force de rupture requise pour séparer l'élément de fixation de piston d'un élément d'entrée en contact du champ opératoire du système de glace chirurgicale.

10. Champ opératoire selon la revendication 9, dans lequel la largeur (134) de la partie de paroi latérale inférieure (128) est telle que la partie de paroi latérale inférieure retient l'élément d'alignement inséré dans la partie latérale (122) pendant le fonctionnement normal de la machine chirurgicale de production de givre.

11. Champ opératoire selon la revendication 10, dans lequel la largeur (134) de la partie de paroi latérale inférieure (128) est telle que l'élément d'alignement tire à travers la partie de paroi latérale inférieure si la force générée entre l'élément de fixation de piston et l'élément d'engagement de Champ opératoire dépasse un seuil.

12. Champ opératoire selon la revendication 11, dans lequel le seuil correspond à une force suffisante pour endommager la machine chirurgicale de production de neige fondante.

13. Champ opératoire selon la revendication 11 ou 12, dans lequel la partie de paroi latérale inférieure (128) est conçue pour se détacher si le seuil est atteint.

14. Champ opératoire selon la revendication 13, dans lequel la partie de paroi latérale inférieure (128) comprend une région affaiblie conçue pour se rompre lors du dépassement du seuil.

15. Champ opératoire selon la revendication 11 ou 12, dans lequel la partie de paroi latérale inférieure (128) est conçue pour se déformer si le seuil est atteint.
